(19) Europäisches Patentamt

European Patent Office

Office européen des brevets

(11) **EP 3 538 046 B1**

(12) **EUROPEAN PATENT SPECIFICATION**

(45) Date of publication and mention of the grant of the patent:
**02.12.2020 Bulletin 2020/49**

(51) Int Cl.:
*A61F 13/56* *(2006.01)*      *A61F 13/551* *(2006.01)*

(21) Application number: **17764961.3**

(22) Date of filing: **29.08.2017**

(86) International application number:
**PCT/US2017/049026**

(87) International publication number:
**WO 2018/089088 (17.05.2018 Gazette 2018/20)**

(54) **ARRAY OF ABSORBENT ARTICLES WITH EAR PORTIONS**

ANORDNUNG AUS SAUGFÄHIGEN ARTIKEL MIT LASCHENTEILEN

ENSEMBLE D'ARTICLES ABSORBANTS MUNIS DE PARTIES DE LANGUETTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.11.2016 US 201662419518 P**
          **11.08.2017 US 201715674559**
          **11.08.2017 US 201715674566**

(43) Date of publication of application:
**18.09.2019 Bulletin 2019/38**

(73) Proprietor: **The Procter & Gamble Company Cincinnati, OH 45202 (US)**

(72) Inventors:
• **MUELLER, Joerg**
  **65824 Schwalbach am Taunus (DE)**
• **DALAL, Urmish, Popatlal**
  **Cincinnati, Ohio 45202 (US)**

(74) Representative: **P&G Patent Germany Procter & Gamble Service GmbH Sulzbacher Straße 40 65824 Schwalbach am Taunus (DE)**

(56) References cited:
**WO-A1-2012/154318**      **US-A1- 2012 238 980**
**US-A1- 2016 206 485**

**Description**

FIELD OF THE INVENTION

The present invention relates to absorbent articles having ear portions, in particular stretchable ears.

BACKGROUND OF THE INVENTION

It has long been known that absorbent articles such as conventional absorbent articles (*e.g.,* diapers, adult incontinence articles, feminine hygiene pads) offer the benefit of receiving and containing urine and/or other bodily exudates (*e.g.,* feces, menses, mixture of feces and urine, mixture of menses and urine, etc.). To effectively contain bodily exudates, the article should provide a snug fit around the waist and legs of a wearer.

Manufacturers often use extensible areas, such as stretch side panels (i.e., ears), within the article to help achieve a snug fit. When worn, the stretch ears extend the article about the hip and waist of the wearer to anchor the product in use while still allowing the wearer to move comfortably. A fastening system is typically joined to the ear to further secure the product about the wearer. Stretch ears are typically laminates of coverstock materials (such as nonwovens) and elastomeric materials.

It has been proposed to create stretch laminates using ultrasonic bonding. In such instance, a stretched elastomeric material is combined with a nonwoven via ultrasonic bonding. After combination, the nonwoven will form corrugations when the laminate is in a relaxed state. These laminates can produce highly stretchable ears (depending on the level of stretch imparted in the elastomeric material) while avoiding the use of glues and mechanical activation. Further, unlike other forms of lamination, the elastomeric material need not extend across the entire width of the laminate. In addition, ultrasonically bonded laminates provide higher levels of breathability than other stretch laminates.

While ultrasonically bonded laminates offer a number of benefits, different executions may be necessary to suit the needs of individual wearers or different segments of consumers. Indeed, absorbent articles are typically offered in different sizes, corresponding to a range of weights for potential wearers. Further, absorbent articles may be provided different features which may correspond to performance (e.g., absorbency, leakage protection, and/or extensibility), comfort (e.g., softness), fit (e.g., extensibility) and/or stages of development of potential wearers (e.g., activity levels, body shape). Manufacturers often provide multiple product offerings in order to serve different consumer segments.

Therefore, there is a need for ultrasonically bonded laminates that are adapted to different product offerings, including different sizes, fit, performance and/or comfort requirements. Further, there is a continued need for stretch ears having desirable stretch balanced with adequate strength. There is also a need for stretch ears having improved breathability while maintaining strength, appropriate stress profiles, stretch profiles, force profiles and/or other desirable properties.

US Patent Application 2012/0238980 describes disposable absorbent articles with ears that may be made from an elastic laminate material and that have elasticized regions that can include ultrasonic bonds.

SUMMARY OF THE INVENTION

The present invention relates to an array of absorbent articles having a first absorbent article and a second absorbent article, as described in claim 1. The first absorbent article i comprises a topsheet, a backsheet and an absorbent core disposed between the topsheet and backsheet, and a first ear laminate having a first plurality of ultrasonic bonds. The second absorbent article comprises a topsheet, a backsheet and an absorbent core disposed between the topsheet and backsheet, and a second ear laminate having a second plurality of ultrasonic bonds. The first and second ear laminates differ at least in bond strength. In some embodiments, the first ear laminate comprises an Average Extension at 10N that is at least 5 % greater than an Average Extension of the second ear laminate at 10N. Additionally, the first plurality of ultrasonic bonds is disposed in a first collective pattern, and the second plurality of ultrasonic bonds is disposed in a second collective pattern. The first and second collective patterns differ by average bond spacing, pattern uniformity, bond size, bond shape, bond orientation, aggregate bond area, aggregate pattern shape and combinations thereof. In further embodiments, the first ear laminate may comprise a TS7 softness value that is at least 10% less than a TS7 softness value of the second ear laminate. The first ear laminate may further comprise an Average Load at Break that is at least 4% greater than an Average Load at Break of the second ear laminate.

BRIEF DESCRIPTION OF THE DRAWINGS

Fig. 1 is schematic plan view of an exemplary absorbent article according to one nonlimiting embodiment of the present invention. The absorbent article is shown in a flat, uncontracted state.

Fig. 2 is a schematic plan view of an exemplary ear according to one nonlimiting embodiment of the present invention.

Fig. 3 is an exploded perspective view of an exemplary ear according to a nonlimiting embodiment of the present invention.

Fig. 4 is a schematic plan view of an exemplary ear according to one nonlimiting embodiment of the present invention.

Fig. 5 is a schematic cross sectional view of the ear in Fig. 4 taken along the ear's lateral centerline.

Fig. 6 is a schematic cross sectional view of an exemplary ear according to another nonlimiting embodiment of the present invention.

Fig. 7 is a schematic plan view of an exemplary ear according to a nonlimiting embodiment of the present invention.

Fig. 8 is a schematic plan view of an array of absorbent articles in accordance with a nonlimiting embodiment of the present invention.

Fig. 9 is a schematic plan view of exemplary ears in accordance with a nonlimiting embodiment of the present invention.

Figs. 10-12 are schematic perspective views of packages in accordance with nonlimiting embodiments of the present invention.

Fig. 13 is a schematic perspective view of grips suitable for use in the Tensile Test Method herein.

Fig. 14 is a schematic side elevation view of a grip suitable for use in the Tensile Test Method herein.

## DETAILED DESCRIPTION OF THE INVENTION

## DEFINITIONS

"Disposable," in reference to absorbent articles, means that the absorbent articles are generally not intended to be laundered or otherwise restored or reused as absorbent articles (i.e., they are intended to be discarded after a single use and, preferably, to be recycled, composted or otherwise discarded in an environmentally compatible manner).

"Absorbent article" refers to devices which absorb and contain body exudates and, more specifically, refers to devices which are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Patent No. 6,120,487), refastenable diapers or pant-type diapers, incontinence briefs and undergarments, diaper holders and liners, feminine hygiene garments such as panty liners, absorbent inserts, and the like.

"Activation" is the mechanical deformation of a plastically extensible material that results in permanent elongation of the extensible material in the direction of activation in the X-Y plane of the material. Activation of a laminate that includes an elastic material joined to a plastically extensible material typically results in permanent deformation of the plastic material, while the elastic material returns substantially to its original dimension. Activation processes are disclosed in U.S. Pat. Pub. No. 2013/0082418, U.S. Pat. No. 5,167,897 and U.S. Patent No. 5,993,432.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Joined" refers to configurations whereby an element is directly secured to another element by affixing the element directly to the other element and to configurations whereby an element is indirectly secured to another element by affixing the element to intermediate member(s) which in turn are affixed to the other element.

"Elastic," "elastomeric," and "elastically extensible" mean the ability of a material to stretch by at least 100% without rupture or breakage at a given load, and upon release of the load the elastic material or component exhibits at least 80% recovery (i.e., has less than 20% set) in one of the directions as per the Hysteresis Test described herein. Stretch, sometimes referred to as strain, percent strain, engineering strain, draw ratio, or elongation, along with recovery and set may each be determined according to the Hysteresis Test described in more detail below. Materials that are not elastic are referred as inelastic.

"Extensible" means the ability to stretch or elongate, without rupture or breakage, by at least 50% as per step 5(a) in the Hysteresis Test herein (replacing the specified 100% strain with 50% strain).

"Film" means a sheet-like material wherein the length and width of the material far exceed the thickness of the material (e.g., 10x, 50x, or even 1000x or more). Films are typically liquid impermeable but may be configured to be breathable.

"Laminate" means two or more materials that are bonded to one another by any suitable method known in the art (e.g., adhesive bonding, thermal bonding, ultrasonic bonding, or high pressure bonding using non-heated or heated patterned roll).

"Lateral" refers to a direction running from a longitudinal edge to an opposing longitudinal edge of the article and generally at a right angle to the longitudinal direction. Directions within 45 degrees of the lateral direction are considered to be "lateral."

"Longitudinal" refers to a direction running substantially perpendicular from a waist edge to an opposing waist edge of the article and generally parallel to the maximum linear dimension of the article. Directions within 45 degrees of the longitudinal direction are considered to be "longitudinal."

"Nonwoven" means a porous, fibrous material made from continuous (long) filaments (fibers) and/or discontinuous (short) filaments (fibers) by processes such as, for example, spunbonding, meltblowing, airlaying, carding, coforming, hydroentangling, and the like. Nonwovens do not have a woven or knitted filament pattern. Nonwovens may be liquid permeable or impermeable.

"Carded fibers" refer to fibers that are of a discrete length which are sorted, separated, and at least partially aligned by a carding process. For example, a carded web refers to a web that is made from fibers which are sent through a combing or carding unit, which separates or breaks apart and aligns the fibers in, e.g., the machine direction to form a generally machine direction-oriented fibrous non-woven web. Carded fibers may or may not be bonded after being carded.

"Meltblown fibers" refers to fibers made via a process whereby a molten material (typically a polymer), is extruded under pressure through orifices in a spinneret or die. High velocity hot air impinges upon and entrains the filaments as they exit the die to form filaments that are elongated and reduced in diameter and are fractured so that fibers of generally variable but mostly finite lengths are produced. This differs from a spunbond process whereby the continuity of the filaments is preserved along their length. An exemplary meltblown process may be found in U.S. Pat. No. 3,849,241 to Buntin et al.

"Spunbond fibers" refers to fibers made via a process involving extruding a molten thermoplastic material as filaments from a plurality of fine, typically circular, capillaries of a spinneret, with the filaments then being attenuated by applying a draw tension and drawn mechanically or pneumatically (e.g., mechanically wrapping the filaments around a draw roll or entraining the filaments in an air stream). The filaments may be quenched by an air stream prior to or while being drawn. The continuity of the filaments is typically preserved in a spunbond process. The filaments may be deposited on a collecting surface to form a web of randomly arranged substantially continuous filaments, which can thereafter be bonded together to form a coherent nonwoven fabric. Exemplary spunbond process and/or webs formed thereby may be found in U.S. Pat. Nos. 3,338,992; 3,692,613, 3,802,817; 4,405,297 and 5,665,300.

"Crimped spunbond fibers" refers to spunbond bi-component fibers which have a helical crimp or curl. The crimped fibers may be configured in a side-by side, core-eccentric sheath or other suitable configuration. The selection of suitable resin combinations and bi-component fiber configurations can lead to the helical crimp or curl generated fibers. Where suitable configurations and/or resin combinations exist, the crimp may occur spontaneously during the spinning or laydown process, on its own after web formation. In some instances, the webs may require an additional step (e.g. heating or mechanical deformation) to induce the fibers to crimp.

"Relaxed" means the state of an element, material or component at rest with substantially no external force acting on the element, other than gravity.

A "segment" refers to actual or potential purchasers and/or wearers having shared characteristics, including but not limited to common needs, common interests, similar lifestyles, and similar demographic profiles, which may cause them to respond to a particular product similarly. Segments may include purchasers/wearers desiring products having a particular performance level, residing in a particular geography, and/or seeking a particular price. By way of nonlimiting example, a first segment may prioritize high performing absorbent articles with little regard to their price while a second segment may prioritize low price absorbent articles and is satisfied with lower performance standards. A first segment may prioritize one feature (e.g., softness), while a second segment may prioritize a different feature (e.g., absorbency). Manufacturers often provide different product offerings based on purchaser and/or wearer segments.

"Design element" as used herein means a shape or combination of shapes that visually create a distinct and discrete component, regardless of the size or orientation of the component. A design element may be present in one or more patterns. A design element may be present one or more times within one pattern. In one nonlimiting example, the same design element is present twice in one pattern - the second instance of the design element is smaller than the first instance. One of skill in the art will recognize that alternative arrangements are also possible. Design elements may comprise insignia. Design elements and/or combinations of design elements may comprise letters, words and/or graphics such as flowers, butterflies, hearts, character representations and the like. Design elements may be formed from bonds, including the shape of one or more bond(s). Design elements and/or combinations of design elements may comprise instructional indicia providing guidance or instruction to the caregiver relative to placement and/or fit of the article about the wearer.

"Pattern" as used herein means a decorative or distinctive design, not necessarily repeating or imitative, including but not limited to the following: clustered, geometric, spotted, helical, swirl, arrayed, textured, spiral, cycle, contoured, laced, tessellated, starburst, lobed, blocks, pleated, concave, convex, braided, tapered, and combinations thereof. In some embodiments, the pattern includes one or more repeating design elements.

"Insignia" as used herein means objects, character representations, words, colors, shapes or other indicia that can be used to distinguish, identify or represent the manufacturer, retailer, distributor and/or brand of a product, including but not limited to trademarks, logos, emblems, symbols, designs, figures, fonts, lettering, crests or similar identifying marks.

"Brand name" means a single source identifier, in other words, a brand name identifies a product and/or service as exclusively coming from a single commercial source (i.e., company). An example of a brand name is PAMPERS®, which is also a trademark. Absorbent articles of the present invention may be marketed and/or packaged under the same brand name. In addition to the brand name, a product descriptor (e.g., Extra Absorbent) or other insignia (e.g., Swaddlers® may also be associated with the absorbent article.

"Bond density" refers to bond frequency and/or aggregate bond coverage.

"Bond frequency" refers to the number of bonds per $cm^2$ as determined by the Bond Dimensions Test Method herein.

"Aggregate bond coverage" refers to the sum of the bond areas in a given region as determined by the Bond Dimension Test Method herein.

ABSORBENT ARTICLE

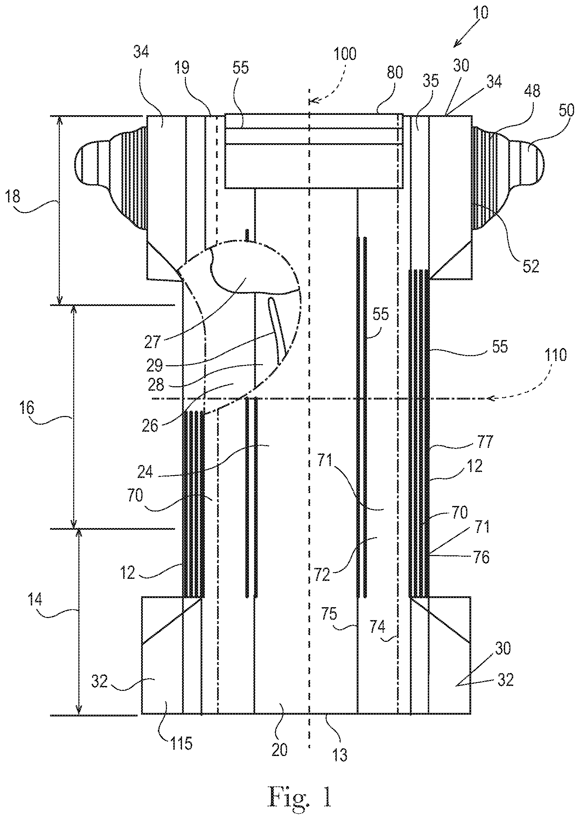

Fig. 1 is a plan view of an exemplary, non-limiting embodiment of an absorbent article 10 of the present invention in a flat, uncontracted state. The body-facing surface 115 of the absorbent article 10 is facing the viewer. The absorbent article 10 includes a longitudinal centerline 100 and a lateral centerline 110.

The absorbent article 10 comprises a chassis 20. The absorbent article 10 and chassis 20 are shown to have a first waist region 14, a second waist region 18 opposed to the first waist region 14, and a crotch region 16 located between the first waist region 14 and the second waist region 18. The waist regions 14 and 18 generally comprise those portions of the absorbent article 10 which, when worn, encircle the waist of the wearer. The waist regions 14 and 18 may include elastic members 55 such that they gather about the waist of the wearer to provide improved fit and containment. The crotch region 16 is the portion of the absorbent article 10 which, when the absorbent article 10 is worn, is generally positioned between the legs of the wearer.

The outer periphery of the chassis 20 is defined by longitudinal edges 12 and waist edges (first waist edge 13 in first waist region 14 and second waist edge 19 in second waist region 18). The chassis 20 may have opposing longitudinal edges 12 that are oriented generally parallel to the longitudinal centerline 100. However, for better fit, longitudinal edges 12 may be curved or angled to produce, for example, an "hourglass" shape article when viewed in a plan view as shown in Fig. 1. The chassis 20 may have opposing lateral edges 13, 19 (i.e., the first waist edge 13 and second waist edge 19) that are oriented generally parallel to the lateral centerline 110.

The chassis 20 may comprise a liquid permeable topsheet 24, a backsheet 26, and an absorbent core 28 between the topsheet 24 and the backsheet 26. The topsheet 24 may be joined to the core 28 and/or the backsheet 26. The backsheet 26 may be joined to the core 28 and/or the topsheet 24. It should be recognized that other structures, elements, or substrates may be positioned between the core 28 and the topsheet 24 and/or backsheet 26. In some embodiments, an acquisition-distribution system 27 is disposed between the topsheet 26 and the absorbent core 28.

In certain embodiments, the chassis 20 comprises the main structure of the absorbent article 10 with other features added to form the composite absorbent article structure. While the topsheet 24, the backsheet 26, and the absorbent core 28 may be assembled in a variety of well-known configurations, absorbent article configurations are described generally in U.S. Patent Nos. 3,860,003; 5,151,092; 5,221,274; 5,554,145; 5,569,234; 5,580,411; and 6,004,306.

TOPSHEET:

The topsheet 24 is generally a portion of the absorbent article 10 that may be positioned at least in partial contact or close proximity to a wearer. Suitable topsheets 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. The topsheet 24 is generally supple, soft feeling, and non-irritating to a wearer's skin. Generally, at least a portion of the topsheet 24 is liquid pervious, permitting liquid to readily penetrate through the thickness of the topsheet 24. One topsheet 24 useful herein is available from BBA Fiberweb, Brentwood, TN as supplier code 055SLPV09U. The topsheet 24 may be apertured.

Any portion of the topsheet 24 may be coated with a lotion or skin care composition as is known in the art. Non-limiting examples of suitable lotions include those described in U.S. Patent Nos. 5,607,760; 5,609,587; 5,635,191; and 5,643,588. The topsheet 24 may be fully or partially elasticized or may be foreshortened so as to provide a void space between the topsheet 24 and the core 28. Exemplary structures including elasticized or foreshortened topsheets are described in more detail in U.S. Patent Nos. 4,892,536; 4,990,147; 5,037,416; and 5,269,775.

ABSORBENT CORE:

The absorbent core 28 may comprise a wide variety of liquid-absorbent materials commonly used in disposable diapers and other absorbent articles. Examples of suitable absorbent materials include comminuted wood pulp, which is generally referred to as air felt creped cellulose wadding; melt blown polymers, including co-form; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials. In one embodiment, at least a portion of the absorbent core is substantially cellulose free and contains less than 10% by weight cellulosic fibers, less than 5% cellulosic fibers, less than 1% cellulosic fibers, no more than an immaterial amount of cellulosic fibers or no cellulosic fibers. It should be understood that an immaterial amount of cellulosic material does not materially affect at least one of the thinness, flexibility, and absorbency of the portion of the absorbent core that is substantially cellulose free. Among other benefits, it is believed that when at least a portion of the absorbent core is substantially cellulose free, this portion of the absorbent core is significantly thinner and more flexible than a similar absorbent core that includes more than 10% by weight of cellulosic fibers. The amount of absorbent material, such as absorbent particulate polymer material present in the absorbent core may vary, but in certain embodiments, is present in the absorbent core in an amount greater than about 80% by weight of the absorbent core, or greater than about 85% by weight of the absorbent core, or greater than about 90% by weight of the absorbent core, or greater than about 95% by weight of the core. In some embodiments, the absorbent core may comprise one or more channels 29, wherein said channels are substantially free of absorbent particulate polymer material. The channels 29 may extend longitudinally or laterally. The absorbent core may further comprise two or more channels. The channels may be straight, curvilinear, angled or any workable combination thereof. In one nonlimiting example, two channels are symmetrically disposed about the longitudinal axis.

Exemplary absorbent structures for use as the absorbent core 28 are described in U.S. Patent No. 4,610,678; 4,673,402; 4,834,735; 4,888,231; 5,137,537; 5,147,345; 5,342,338; 5,260,345; 5,387,207; 5,397,316, and U.S. Patent App. Nos. 13/491,642 and 15/232,901.

BACKSHEET:

The backsheet 26 is generally positioned such that it may be at least a portion of the garment-facing surface of the absorbent article 10. Backsheet 26 may be designed to prevent the exudates absorbed by and contained within the absorbent article 10 from soiling articles that may contact the absorbent article 10, such as bed sheets and undergarments. In certain embodiments, the backsheet 26 is substantially water-impermeable. Suitable backsheet 26 materials include films such as those manufactured by Tredegar Industries Inc. of Terre Haute, IN and sold under the trade names X15306, X10962, and X10964. Other suitable backsheet 26 materials may include breathable materials that permit vapors to escape from the absorbent article 10 while still preventing exudates from passing through the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable composite materials comprising polymer blends are available from Clopay Corporation, Cincinnati, OH under the name HYTREL blend P18-3097. Suitable breathable backsheets and breathable composite materials are described in greater detail in PCT App. No. WO 95/16746; U.S. Patent No. 5,865,823; U.S. Patent No. 5,571,096; and U.S. Patent No. 6,107,537. Other suitable materials and/or manufacturing techniques may be used to provide a suitable backsheet 26 including, but not limited to, surface treatments, particular film selections and processing, particular filament selections and processing, etc.

Backsheet 26 may also consist of more than one layer. The backsheet 26 may comprise an outer cover and an inner layer. The outer cover may be made of a soft, non-woven material. The inner layer may be made of a substantially liquid-impermeable film, such as a polymeric film. The outer cover and an inner layer may be joined together by adhesive or any other suitable material or method. A particularly suitable outer cover is available from Corovin GmbH, Peine, Germany as supplier code A18AH0, and a particularly suitable inner layer is available from RKW Gronau GmbH, Gronau, Germany as supplier code PGBR4WPR. While a variety of backsheet configurations are contemplated herein, it would be obvious to those skilled in the art that various other modifications can be made without departing from the spirit and scope of the invention.

EARS/FASTENERS:

The absorbent article 10 may include one or more ears 30, including for example front ears 32 disposed in the first waist region and/or back ears 34 disposed in the second waist region. The ears 30 may be integral with the chassis or discrete elements joined to the chassis 20 at a chassis attachment bond 35, which may join one or more layers of the ear to the chassis. The ears 30 may be extensible or elastic. The ears 30 may be formed from one or more nonwoven webs, woven webs, knitted fabrics, polymeric and elastomeric films, apertured films, sponges, foams, scrims, or combinations and/or laminates of any the foregoing.

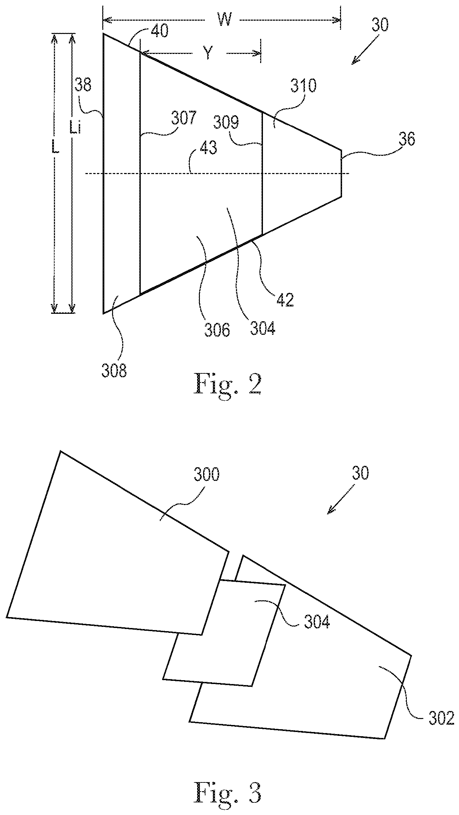

As illustrated in Fig. 2, ears may include an outboard edge 36 and an inboard edge 38. The outboard edge 36 is the free distal longitudinal edge of the ear when said ear is joined to the chassis 20. The inboard edge 38 is substantially opposed to the outboard edge and is joined to or overlapped with the chassis when the ear is joined to the chassis or is the side defined by a line extending from longitudinal side 12 in the widest area of the crotch region and running parallel to the longitudinal centerline in the case of integral ears. The inboard edge comprises a length, Li. Ears may further include a first lateral side 40 and an opposing second lateral side 42, and lateral centerline line 43 which is generally parallel to the article's lateral centerline 110 when the ear is joined to the article. An ear may additional comprise a maximum width, W, extending between the outboard edge and inboard edge and a maximum length, L, extending between the first and second lateral sides. In some embodiments, the maximum length is the length of the inboard edge, Li.

In some embodiments, the ear 30 may include elastomers, such that the ear is stretchable. In certain embodiments, the ears 30 may be formed of a stretch laminate such as a nonwoven/elastomeric material laminate or a nonwoven/elastomeric material/nonwoven laminate, which also results in the ear being stretchable. The ear 30 may be extensible in the lateral direction. In some embodiments, the ear is elastic in the lateral direction. In further embodiments, the ear 30 may extend more in the lateral direction than in the longitudinal direction. Alternatively, the ear may extend more in the longitudinal direction than in the lateral direction.

In some embodiments, the ear comprises a laminate of a nonwoven 300 and an elastomeric layer 304. In certain embodiments illustrated in Fig. 3, an ear comprises a body-facing nonwoven 300, a garment-facing nonwoven 302 and an elastomeric layer 304. The elastomeric layer 304 may be sandwiched between the body-facing and garment-facing nonwovens. Additional layers may be included (e.g., additional nonwovens, inelastic materials, elastic or extensible materials, etc.).

Any suitable nonwoven may be used in an ear 30. Suitable nonwovens may comprise a basis weight of at least about 8 gsm, or about 30 gsm or less, or about 22 gsm or less, or about 20 gsm or less, or about 17 gsm or less, or from about 10 gsm to about 22 gsm, reciting for said range every 1 increment therein. Typically, lower basis weight nonwovens reduce an ear's collective strength. However, the inventors have discovered ears designed according to the principles herein can obtain high strength despite lower basis weight nonwovens.

A nonwoven may comprise meltblown layers, carded layers, and/or spunbond layers. In a nonlimiting example, a nonwoven comprises two or more spunbond layers. In further nonlimiting examples, one or more nonwovens may comprise a SMS configuration. Alternatively, one or more of the nonwovens in the ear may be void of meltblown layers. While meltblown layers have been found to enhance bonding in ears requiring adhesive (given the meltblown layer's inhibition of the adhesive's diffusion through the porous nonwoven structure), meltblown layers often lack strength. In some embodiments, a nonwoven consists essentially of spunbond layers. In some nonlimiting examples, both the body-facing and the garment-facing nonwoven comprises at least 2 spunbond layers, or 3 or more spunbond layers. In some embodiments, one or more nonwovens in the ear laminate comprises crimped spunbond fibers.

Nonwoven softness is often associated with tactile feel. Sleek or silky feel is often preferred over rough texture. Various approaches can be used to deliver silky feel. A nonwoven web can be made of bi-component or multi-component fibers. One of the components of the fibers, preferably outer component, is soft polymer such as polyethylene or elastic polyolefin, polyurethane. For example, in sheath/core bi-component fiber, sheath can be made of polyethylene while core can be made of polypropylene. Alternatively, a nonwoven web can be made of mono-component fiber with a polymer blend that imparts softness, such as polypropylene blended with elastomeric polypropylene (VISTAMAXX® from Exxon).

A nonwoven web can be made of fibers comprising elastomeric polymer, such as elastomeric polyolefins. Additionally or alternatively, additives can be added to polymer before spinning fiber. During fiber spinning and subsequent process steps to make nonwoven web, the additives migrate to fiber surface to provide silky feel. Amine and Amide based additives are commonly used up to 5% to impart softness.

In another approach, sleek chemical finish can be coated on the fibers or nonwoven webs. Chemical finishes based on oil, silicone, esters, fatty acids, surfactant etc. can be employed. Softness additives such as anionic, cationic or nonionic can also be used to improve drape, and touch. Various coating techniques, like roll coating, screen coating, gravure coating, slot coating, spray coating, can be used to apply finish.

In another approach, nonwoven fiber diameter can be reduced to produce fine fibers and to provide silk like feel. Meltblown fiber is one technology to reduce fiber diameter to less than 20 microns. Alternatively, nanofibers, having a diameter of less than 1 micron, made from a melt film fibrillation process with a polymer composition disclosed in US8835709 patent can be used to provide softness.

Bending or pliability of material without any external force and under its own weight communicates softness. It can be influenced by variety of factors such as fiber chemistry, thickness, nonwoven bond pattern. Pliability or drape is linked to bending stiffness, which is related to inherent elastic modulus and thickness of material. It has proven to be advantageous for the nonwoven fabric to have a minimum and a maximum bending stiffness, since for instance in the use of the nonwoven fabric in contour matching, as in medical and hygiene articles, too stiff a material would be undesirable. Polyolefin resin with lower elastic modulus and/or lower crystallinity enables lower bending stiffness. One can blend lower elastic modulus materials (elastomer) with traditional fiber making polyolefin resin to make lower modulus fibers. Optimizing bonding can also alter the bending stiffness of the web in the direction desired. Bonds with larger aspect ratio of longitudinal dimension to lateral dimension provides better drape in lateral dimension while providing right rigidity and strength for web handling. Another factor affecting drape is the thickness of the web. The thicker the web is, the lower is the flexibility or pliability. Combining right thickness with fiber chemistry or bond pattern, better drape can be achieved while delivering web performance suitable for processing.

Where the ear 30 comprises more than one nonwoven, the nonwovens may comprise the same basis weight or different basis weights. Likewise, the nonwovens may comprise the same layer structure (e.g., SMS) or different layer structures (e.g., SS, SMS). Further, a nonwoven in the ear may comprise the same or different features of nonwovens in the backsheet, topsheet, leg gasketing system and/or waist feature.

The elastomeric layer 304 comprises one or more elastomeric materials which provide elasticity to at least a portion of the layer 304. Nonlimiting examples of elastomeric materials include film (e.g., polyurethane films, films derived from rubber and/or other polymeric materials), an elastomeric coating applied to another substrate (e.g., a hot melt elastomer, an elastomeric adhesive, printed elastomer or elastomer co-extruded to another substrate), elastomeric nonwovens, scrims, and the like. Elastomeric materials can be formed from elastomeric polymers including polymers comprising styrene derivatives, polyesters, polyurethanes, polyether amides, polyolefins, combinations thereof or any suitable known elastomers including but not limited to co-extruded VISTAMAXX®. Exemplary elastomers and/or elastomeric materials are disclosed in U.S. Pat. Nos. 8,618,350; 6,410,129; 7,819,853; 8,795,809; 7,806,883; 6,677,258 and U.S. Pat. Pub. No. 2009/0258210. Commercially available elastomeric materials include KRATON (styrenic block copolymer; available from the Kraton Chemical Company, Houston, TX), SEPTON (styrenic block copolymer; available from Kuraray America, Inc., New York, NY), VECTOR (styrenic block copolymer; available from TSRC Dexco Chemical Company, Houston, TX), ESTANE (polyurethane; available from Lubrizol, Inc, Ohio), PEBAX (polyether block amide; available from Arkema Chemicals, Philadelphia, PA), HYTREL (polyester; available from DuPont, Wilmington, DE), VISTAMAXX (homopolyolefins and random copolymers, and blends of random copolymers, available from EXXON Mobile, Spring, TX) and VERSIFY (homopolyolefins and random copolymers, and blends of random copolymers, available from Dow Chemical Company, Midland, Michigan).

In nonlimiting examples, the elastomeric layer 304 comprises a film. The film may comprise a single layer or multiple layers. The film may be extensible in the lateral direction or may be elastic in the lateral direction. The film may be preactivated as disclosed, for example, in U.S. Patent No. 9,533,067. The elastomeric layer may comprise a width, Y, as shown for example in Fig. 2. In some embodiments, Y is less than the width, W, of the ear 30 by at least about 10 mm. The elastomeric layer may have a longitudinal dimension that is the same as the ear 30 along with the width of the elastomeric layer, or a longitudinal dimension that is less than the longitudinal length of the ear at any point along with the width of the elastomeric layer. In some embodiments, the elastomeric layer may have a basis weight of from about 5 to about 150 gsm, or from about 10 to about 100 gsm, or less than about 150 gsm, reciting for each range every 5 gsm increment therein.

As also illustrated in Fig. 2, the ear 30 may comprise an elasticized region 306. The elasticized region 306 is generally defined by the perimeter of the elastomeric material 304. In the elasticized region 306, the ear is elastically extensible. In some embodiments, the area of the elasticized region comprises at least about 20% of, or from about 30% to about 100%, or about 80% or less of the total area of the ear, reciting for said range every 5% increment therein. In further embodiments, Y (i.e., the maximum width of the elastomeric layer) is at least about 20% of, or from about 25% to about 100%, or from about 35% to about 85%, or about 80% or less of the total width, W, of the ear, reciting for each range every 5% increment therein.

The ear may further comprise one or more inelastic regions. In certain embodiments, the ear 30 comprises a first inelastic region 308, which extends laterally outward from the inboard edge 38 and is adjacent to the elasticized region 306 at a first elastomeric edge 307. The ear may further include a second inelastic region 310, which may extend laterally inward from the outboard edge 36 and may be adjacent to the elasticized region 306 at a second elastomeric edge 309. The first and second inelastic regions may be made of the same material(s) or different materials.

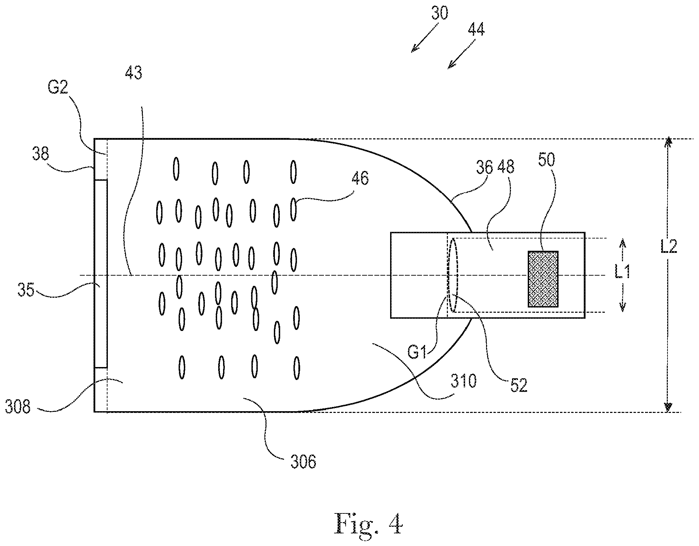

Turning to Fig. 4, in certain embodiments, the ear 30 comprises a gathered laminate 44, wherein one of the layers is strained to a greater degree than a remaining layer during lamination. In this way, the less extensible layer (i.e., the nonwoven 300, 302) will form gathers when the laminate 44 is in a relaxed state. In some embodiments, at least a portion of the elastomeric layer is strained while the nonwoven(s) are in a relaxed state during lamination. The elastomeric layer may be stretched one or more directions. Corrugations then form in the nonwoven layer(s) when the subsequently formed laminate 44 is in a relaxed state. In nonlimiting examples, the elastomeric layer is stretched in a direction corresponding with the lateral direction of the article. In other words, when the ear is joined to the chassis subsequent to lamination, the ear laminate will be oriented such that the ear is stretchable in the lateral direction of the article. In further nonlimiting examples, the ear is also stretchable in the longitudinal direction.

The laminate layers may be joined by one or more ultrasonic bonds 46 as illustrated in Fig. 4. The ultrasonic bonds may join the nonwoven layers through the elastomeric layer. The ultrasonically bonded laminate may be formed by the process and/or equipment disclosed in commonly assigned U.S. Patent App. Nos. 62/374,010 and 62/419,515.

In some embodiments, the laminate may be void of adhesive. In some nonlimiting examples, the ear comprises adhesive bond(s) only at the chassis attachment bond 35 and/or at the fastener attachment bond 52 (discussed below). The fastener attachment bond and/or the chassis attachment bond 35 may comprise ultrasonic bonds and/or may be void of adhesive.

The absorbent article 10 may also include a fastening system 48. When fastened, the fastening system 48 interconnects the first waist region 16 and the rear waist region 18 resulting in a waist circumference that may encircle the wearer during wear of the absorbent article 10. The fastening system 48 may comprise a fastening elements 50 such as tape tabs, hook and loop fastening components, interlocking fasteners such as tabs & slots, buckles, buttons, snaps, and/or hermaphroditic fastening components, although any other known fastening means are generally acceptable. The absorbent article may further comprise a landing zone to which a fastening element can engage and/or a release tape that protects the fastening elements from insult prior to use. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. In some embodiments, the fastening system 48 and/or the element 50 is foldable.

The fastening system 48 may be joined to any suitable portion of the article 10 by any suitable means. In some embodiments, the fastening system is joined to the ear 30 at a fastener attachment bond 52 as illustrated in Figs. 4-6. The fastening system may be joined to the ear between layers. The fastening system may be joined to the ear on an exterior surface as shown for example in Fig. 4. The fastening system and/or fastening elements may be ultrasonically bonded to the ear. The fastening attachment bond 52 comprises a maximum length, L1, measured parallel to the longitudinal centerline. The maximum length may be about 30 mm or less, or about 28 mm or less, or from about 20 mm to about 35 mm, reciting for said range every 1 mm increment therein.

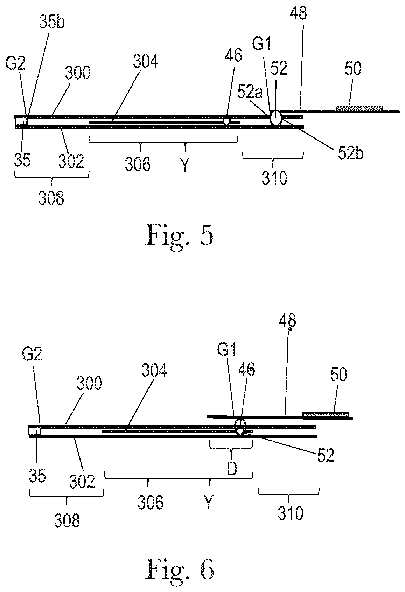

Returning to Figs. 4-6, the fastening system 48 may be joined to ear proximate to the outboard edge 36. The fastening system may be disposed in the second inelastic region 310. In further embodiments, the fastening system 48 is joined in the elasticized region 306 of the ear. The inventors have found that joining the fastening system to the ear in the elasticized region 306 improves the collective strength of the ear/fastening system combination during use and/or application. Without being bound by theory, it is believed that breakage in ears formed from ultrasonically bonded laminates initially occurs in an inelastic region near the outboard edge 36 as the intact nonwoven resists the stretching of the elastomeric layer; and therefore, joining the fastening system within the elasticized region 306 reduces the stress on the inelastic portion of the ear. In some embodiments, the fastening system 48 is joined in the elasticized region such that it overlaps with the elasticized region for a maximum lateral overlap distance of D as depicted in Fig. 6. In certain nonlimiting examples, D may be from about 0.05% to about 5%, or about 1% to about 5% of Y (i.e., the maximum width of the elasticized region), reciting for each range every 0.02% increment therein.

In further embodiments, the ear comprises a Length Ratio of about 3 or less, or about 2.95 or less, or from about 1 to about 3, or from about 1.75 to about 3, or from about 1 to about 2.5 as determined by the Tensile Test Method herein, reciting for each range every .05 interval therein. Forming an ear with such Length Ratios decreases the potential for roping within the ear. Further, the specified Length Ratios result in increased strength in the ear.

The ear may comprise an Average Load at Break of 15 N or greater, or 20 N or greater, or 25 N or greater, 30 N or greater, or 40 N or greater, or from about 15 N to about 45 N, or about 25 N to about 40 N according to the Tensile Test Method herein, reciting for said range every 1 N increment therein. The specified Average Load at Break values may be obtained even when garment-facing and body-facing nonwovens comprise a basis weight of about 17 gsm or less, or about 14 gsm or less, or about 12 gsm or less, or from about 8 gsm to about 17 gsm, reciting for said range every 1 increment therein. Once joined to the ear, the fastening system 48 may comprise an Average Load at Break of 24 N or greater, or about 30 N or greater, or from about 17 N to about 40 N, according to the Tensile Test Method herein, reciting for said range every 1 N increment therein. The specified Average Load at Break values may be obtained even when the body-facing and/or garment-facing nonwovens comprise a basis weight of about 17 gsm or less, or about 14 gsm or less, or about 12 gsm or less, or from about 8 gsm to about 17 gsm, reciting for said range every 1 gsm increment therein.

In other nonlimiting examples, the ultrasonically bonded ear laminate comprises an Average Extension at 5N of about or 10 mm about or greater, or about 15 mm or greater, from about 10 mm to about 25 mm according to the Tensile Test Method herein; and/or an Average Extension at 10N of about 35 mm or greater, about 40 mm or greater, or about 45 mm or greater, or from about 35 mm to about 50 mm according to the Tensile Test Method herein.

In certain embodiments, the ear may comprise an Air Permeability Value of at least about $1m^3/m^2/min$, or from about $2m^3/m^2/min$ to about $125m^3/m^2/min$, or from about $5m^3/m^2/min$ to about $50m^3/m^2/min$ according to the Air Permeability Test Method herein, reciting for each range every $1m^3/m^2/min$ increment therein.

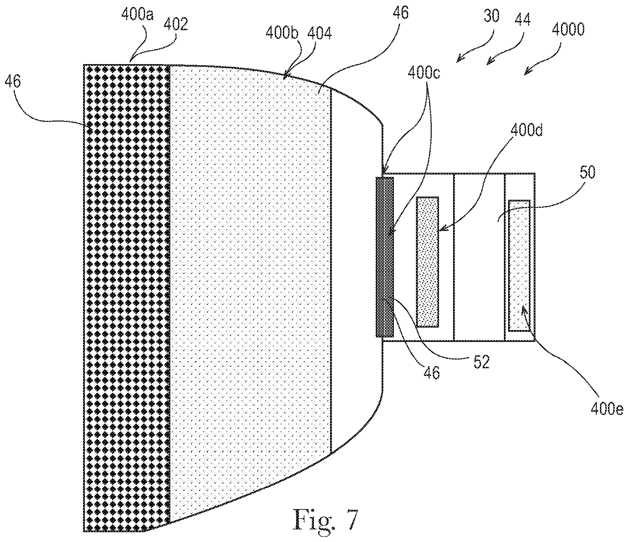

The ear may comprise one or more bond patterns 400. A pattern may be comprised of a plurality of ultrasonic bonds 46. Where the ear comprises multiple bond patterns as in Fig. 7, two or more patterns 400a, 400b may be the same and/or two or more patterns may be different. Patterns may be disposed in different regions of the ear, for example a first bonding region 402 may at least partially overlay an inelastic region which may comprise a different pattern than a second bonding region 404 at least partially overlapping the elasticized region. Other portions of the ear or fastener may comprise different bonding regions, which may comprise patterns 400c, 400d, 400e. The bonding regions may comprise different bond densities, or two or more of the regions may comprise the same bond density while having different bond patterns. In nonlimiting examples, two bonding regions have the same bond density and the two bond patterns differ by the design elements; average spacing between bonds within the patterns; uniformity within the patterns; the sizes, shapes and/or orientation of the bonds within the patterns; the aggregate bond area within the patterns; and/or the collective pattern shape (i.e., the perimeter of the pattern). Additionally or alternatively, two bond patterns may be provided with distinct visual characteristics, including but not limited to insignia, instructional indicia, garment-like patterns and/or other design elements which distinguish one bonding region from one another. In other embodiments, bonding regions comprise substantially the same bond pattern. In some embodiments, bond patterns in different bonding regions but may match, meaning the patterns may comprise substantially similar design elements, which may be rotated, mirrored, reduced in size, enlarged in size and/or altered in aspect ratio between the patterns. Further, where the ear comprises multiple bond patterns and bonding regions, said patterns and regions collectively comprise a collective bond pattern 4000.

LEG GASKETING SYSTEM

Returning to Fig. 1, the absorbent article 10 may comprise a leg gasketing system 70 attached to the chassis 20, which may comprise one or more cuffs 71. The leg gasketing system may comprise a pair of barrier leg cuffs 72. Each barrier leg cuff may be formed by a piece of material which is bonded to the absorbent article so it may extend upwards from a wearer-facing surface of the absorbent article and provide improved containment of fluids and other body exudates approximately at the junction of the torso and legs of the wearer. The barrier leg cuffs are delimited by a proximal edge joined directly or indirectly to the topsheet 24 and/or the backsheet 26 and a free terminal edge 75, which is intended to contact and form a seal with the wearer's skin. In some embodiments, the free terminal edge 75 comprises a folded edge. The barrier leg cuffs 72 extend at least partially between the front waist edge 13 and the rear waist edge 19 of the absorbent article on opposite sides of the longitudinal centerline 100 and are at least present in the crotch region. The barrier leg cuffs may be joined at the proximal edge with the chassis of the article by a bond which may be made by gluing, fusion bonding, or a combination of other suitable bonding processes.

The barrier leg cuffs may be integral with the topsheet 24 or the backsheet 26 or may be a separate material joined to the article's chassis. Each barrier leg cuff 72 may comprise one, two or more elastic elements 55 close to the free terminal edge 75 to provide a better seal.

In addition to the barrier leg cuffs 72, the article may comprise gasketing cuffs 76, which are joined to the chassis of the absorbent article, in particular to the topsheet 24 and/or the backsheet 26 and are placed externally relative to the barrier leg cuffs 72. The gasketing cuffs 76 may provide a better seal around the thighs of the wearer. A gasketing cuff may comprise a proximal edge and a free terminal edge 77. The free terminal edge 77 may comprise a folded edge. Each gasketing cuff may comprise one or more elastic elements 55 in the chassis of the absorbent article between the topsheet 24 and backsheet 26 in the area of the leg openings. All, or a portion of, the barrier leg cuffs and/or gasketing cuffs may be treated with a lotion or another skin care composition.

In further embodiments, the leg gasketing system comprises barrier leg cuffs that are integral with gasketing cuffs. Suitable leg gasketing systems which may be part of the absorbent article are disclosed in U.S. Pat. App. No. 62/134,622, 14/077,708; U.S. Pat. Nos. 8,939,957; 3, 860,003; 7,435,243; 8,062,279.

ELASTIC WAIST FEATURE

The absorbent article 10 may comprise at least one elastic waist feature 80 that helps to provide improved fit and containment, as shown in Fig. 1. The elastic waist feature 80 is generally intended to expand and contract to dynamically fit the wearer's waist. Elasticized waist features include waistbands, waist cuffs having pockets formed from a portion of the waist feature 80 that is unattached from the chassis 20, and waist panels designed to fit securely about the abdomen of the wearer. Nonlimiting examples of elasticized waist features are disclosed in U.S. Patent App. Nos. 13/490,543; 14/533,472; and 62/134,622. Waist features 80 may be joined to the chassis 20 in the first waist region 14 and/or in the second waist region 16. The waist feature can be used in conjunction with the ear 30 to provide desirable stretch and flexibility for proper fit of the article on the wearer.

ARRAY OF ABSORBENT ARTICLES

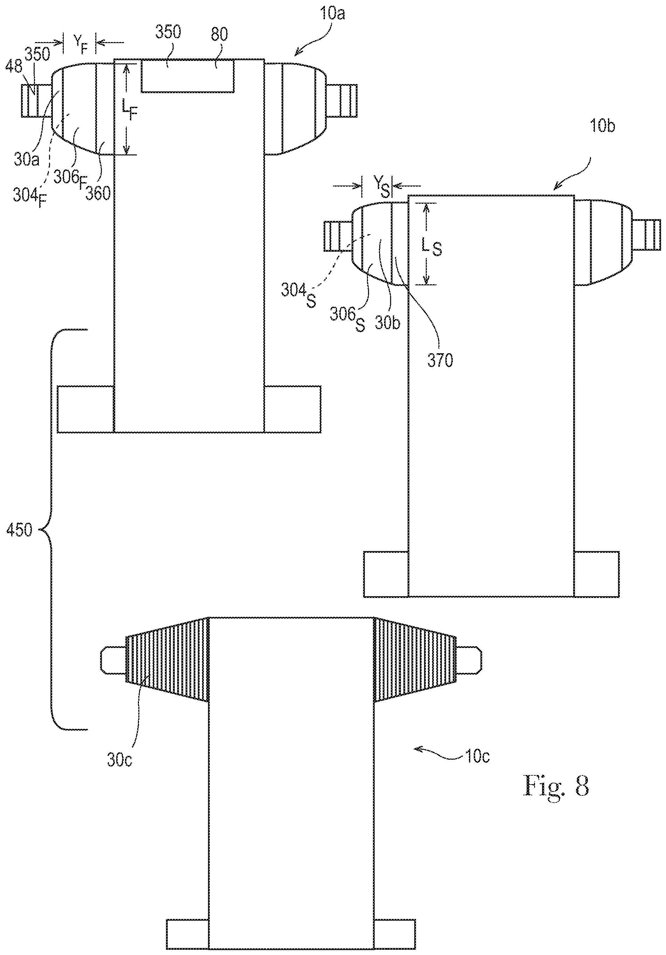

The present invention includes an array 450 of two or more absorbent articles 10a, 10b, 10c as exemplified in Fig. 8. Absorbent articles in the array may be manufactured and/or distributed by a single manufacturer, under a common brand name and/or under a common tradename or trademark. In certain embodiments, the array comprises absorbent articles of different sizes. Additionally or alternatively, the array may comprise absorbent articles suited for different wearer and/or purchaser segments. Each absorbent article comprises a topsheet, a backsheet, an absorbent core between said topsheet and backsheet, and an ear comprising a laminate; each component having any of the features described above relating to the respective components. Two or more ears within the array comprise ultrasonically bonded ear laminates, each comprising a plurality of ultrasonic bonds, and/or two or more ears may comprise a gathered laminate. Absorbent articles in the array may further comprise other features 350 such as fasteners, waist features, wetness indicators and/or leg cuffs.

According to the invention, the array comprises a first absorbent article 10a having a first ear laminate 30a and a second absorbent article 10b having a second ear laminate 30b. Both of the first and second ear laminates each comprise an ultrasonically bonded ear laminate. The first and second ear laminate may each comprise a gathered laminate. Each of the first and second ear laminate may comprise a laminate of a nonwoven layer and an elastomeric layer, as discussed above. The first ear laminate may comprise a first garment-facing nonwoven, a first body-facing nonwoven and a first elastomeric material disposed between said nonwovens. Likewise, the second ear laminate may comprise a second garment facing nonwoven, a second body-facing nonwoven, and a second elastomeric material disposed between said second nonwovens. In some embodiments, the first and second ear laminates are each disposed in the respective second waist regions of the first and second absorbent article; each comprising a back ear.

The first and second ear laminates differ in bond pattern. Additional nonlimiting examples of differences between the ear laminates include differences in extensibility, softness, tensile strength, component materials (e.g., nonwoven or film materials), shape, size, position of the ear on the chassis and combinations thereof. In nonlimiting examples, the first ear laminate and second ear laminate are different sizes. The first ear laminate may comprise a first maximum length, $L_F$, and the second ear laminate may comprise a second maximum length, Ls, which may be different than the first maximum length. In nonlimiting examples, the first maximum length may be at least about 6% greater, or at least about 10% greater, or at least about 12% greater, or at least about 15% greater, or from about 5% to about 50% greater than the second maximum length, reciting for said range every 1% increment therein. In additional nonlimiting examples, the first ear laminate comprises a first area (which is the two-dimensional area of the first ear) and the second ear laminate comprises a second area (i.e., the two-dimensional area of the second ear). The first area may be at least about 6% greater, or at least about 10% greater, or at least about 15% greater, or from about 5% to about 50% greater than the second area. Additionally or alternatively, the width of two ear laminates may differ. It is contemplated that the length of the first ear laminate may be greater than the length of the second ear laminate, and the width of the first ear laminate may be less than the width of the second ear laminate or vice versa.

In an embodiment, the first ear laminate 30a is more extensible than the second ear laminate. For example, the first ear laminate may comprise an Average Extension at 10N that is at least about 5% greater, or about 10% greater, or about 20% greater, or from about 5% to about 30% greater than the Average Extension of the second ear laminate at 10N, reciting for said range every 5% increment therein. In other embodiments, the first ear laminate may comprise an Average Extension at 5N that is at least about 5% greater, or about 10% greater, or about 20% greater, or from about 5% to about 30% greater than the Average Extension of the second ear laminate at 5N, reciting for said range every 5% increment therein. Extensibility at 10N and 5N can be determined by the Tensile Test Method herein.

In certain embodiments, extensibility differences may be provided by differences in the elasticized regions of the ear laminates. In nonlimiting examples, the first ear laminate comprises a first elasticized region $306_F$ and the second ear laminate comprises a second elasticized region 306s, where the second elasticized region comprises one or more dimensions that are different than the first elasticized region. The first elasticized region may comprise a first elasticized area (i.e., the two dimensional area of $306_F$) and the second elasticized region may comprise a second elasticized area (i.e., the two dimensional area of 306s), wherein the first elasticized area may be greater than the second elasticized area. In nonlimiting examples, the first elasticized area is at least about 5%, or least about 10%, or from about 5% to about 30% greater than the second elasticized area. In further nonlimiting examples, the maximum width, $Y_F$, of the first elasticized region may be greater than the maximum width of the second elasticized region, Ys. Additionally or alternatively, the first elasticized region may comprise a first elastomeric material $304_F$, and the second elasticized region may comprise a second elastomeric material 304s. The first and second elastomeric material may be different. For example, the elastomeric materials may differ by basis weight, type of elastic material (e.g., film versus elastic strands), the composition or base materials forming the elastomeric material (e.g. polyurethane films, styrenic film materials, etc.)

and combinations thereof. In nonlimiting examples, the first and second ear laminate comprise gathered laminates and the first elastomeric material is strained to a greater degree during lamination than the second elastomeric material. The first elastomeric material may be strained by at least about 5% more, or at least about 10% more, or from about 5% to about 30% more than the second elastomeric material during the respective first and second ear laminations. In some nonlimiting examples, both the first and the second elastomeric materials comprise film, although the films may differ.

In further nonlimiting examples, the characteristics of one or more nonwovens in the ear laminate may contribute to extensibility and other properties. As stated above, the first ear laminate may comprise a first garment-facing nonwoven and/or a first body-facing nonwoven, and the second ear laminate may comprise a second garment-facing nonwoven and/or a second body-facing nonwoven. One or both of the first garment-facing and first body-facing nonwovens may comprise a primary nonwoven 360. Likewise, one or both of the second garment-facing and second body-facing nonwovens may comprise a secondary nonwoven 370. The primary nonwoven may differ from the secondary nonwoven by one of the group consisting of basis weight, layer configuration (e.g., SMS, carded, SS), fiber composition, fiber configurations (e.g., mono-component or bi-component), fiber denier, fiber diameter, calendar bond area, calendar bond shape, and combinations thereof. These factors may result in differences in extensibility and/or differences in softness.

The first ear laminate and the second ear laminate will each comprise TS7 and TS750 values determinable by the Softness Test Method herein. Lower TS7 and TS750 values indicate greater softness, which is highly desirable in absorbent articles. Wearers and caregivers may find absorbent articles with high TS7 and TS750 values uncomfortable and/or scratchy or otherwise undesirable. In some embodiments, the first ear laminate may comprise a different TS7 value and/or a different TS750 value than the second ear laminate. In nonlimiting examples, the TS7 value of the first ear laminate is lower than the TS7 value of the second ear laminate by least about 10%, or about 15%, or at least about 20%, or from about 5% to about 25%, reciting for said range every 1% increment therein. Additionally or alternatively, the TS750 value of the first ear laminate may be less than the TS750 value of the second ear laminate by least about 10%, or about 15%, or at least about 20%, or from about 5% to about 25%, reciting for said range every 1% increment therein. The TS7 and/or TS750 values may be affected by the type of nonwoven materials and/or elastomeric materials used in the ear laminates. In nonlimiting examples, the ear laminate comprising a low TS7 or TS750 value may comprise a nonwoven having layers comprising component fibers, fibers derived from elastomeric polyolefins and combinations thereof.

The first and second ear laminates may further differ in breathability. In some embodiments, the first ear laminate comprises a first Air Permeability Value and the second ear laminate may comprise a second Air Permeability Value. The first Air Permeability Value may be greater than the second Air Permeability Value by at least about 5%, or at least about 10%, or at least about 20%, or at least about 50%, or from about 5% to about 75%, reciting for said range every 10% increment therein. In nonlimiting examples, the air permeability may be affected by the number of bonds, bond arrangements, and/or bond sizes. In further nonlimiting examples, the array may further comprise an ear laminate that is substantially non-breathable.

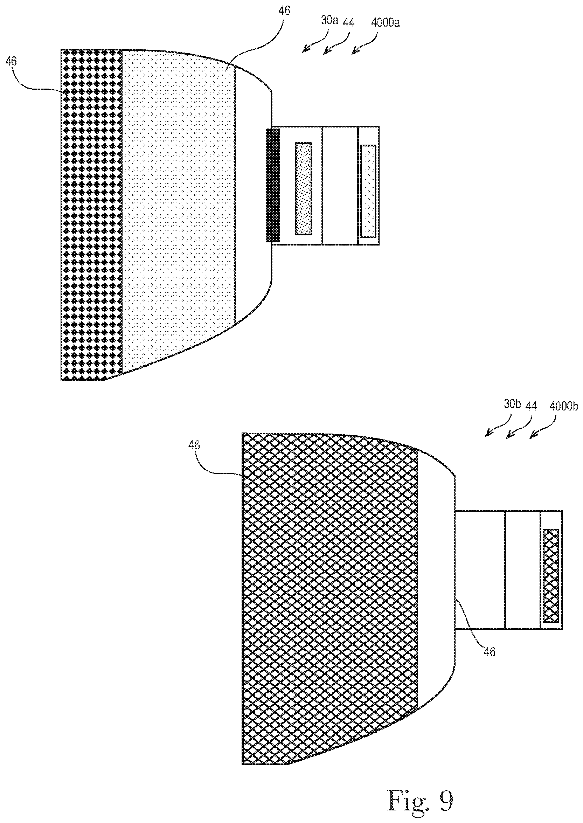

Turning to Fig. 9, the first and second ear laminates comprise different bond patterns or different combinations of bond patterns. The first ear laminate comprises a first collective bond pattern 4000a, which is the combination of the various bond patterns and arrangements within the ear. The second ear laminate comprises a second collective bond pattern 4000b. The first and second collective bond patterns may differ by the average spacing between bonds within the patterns; uniformity within the patterns; the sizes, shapes and/or orientation of the bonds within the patterns; the aggregate bond area within the patterns; and the aggregate pattern shapes (i.e., the perimeters of the collective patterns), and combinations thereof. Differences in bond patterns may result in changes in breathability, extensibility, modulus, tensile strength and/or aesthetic design. In nonlimiting examples, one or more patterns on the first ear laminate matches one or more patterns on the second ear laminate. Additionally or alternatively, patterns on an ear laminate may match patterns on other components of the absorbent article, such as the patterns visible when viewing the topsheet, patterns visible when viewing the backsheet, patterns on fastening systems and/or patterns on leg cuffs. The first and/or second collective pattern may comprise instructional indicia, graphics, and/or insignia. The first collective pattern and/or the second collective pattern may correspond to a user characteristic such as the intended gender of the wearer, intended age and/or development stage, absorbency needs, fit preferences and combinations thereof. The first and second collective patterns may correspond to different user characteristics (e.g., different ages, different absorbency needs).

The first and second ear laminates may further comprise different tensile strength, as determined by the Average Load at Break in the Tensile Test Method herein. In some embodiments, the first ear laminate comprises a first Average Load at Break and the second ear laminate comprises a second Average Load at Break. The first Average Load at Break may be at least about 4% greater, or at least about 10% greater, or at least about 15% greater, or from about 4% to about 25% greater than the second Average Load at Break, reciting for said range every 1% increment therein. The ear laminates may comprise nonwovens having a basis weight of about 30 gsm or less, or about 22 gsm or less, or about 17 gsm or less, or about 14 gsm or less, or about 12 gsm or less, or from about 8 gsm to about 30 gsm, or from about 10 to about 17 gsm, reciting for each range every 1 gsm increment therein.

For the avoidance of doubt, except where property values are reported in percentage or ratios, percent differences for a given property can be calculated by utilizing the respective test method to determine the property values, then using the following formula:

$$\Delta Property = \frac{Property\ for\ First\ Ear - Property\ for\ Second\ Ear}{Property\ for\ Second\ Ear} \times 100\%$$

Where property values are reported in percentages or ratios (e.g., Aggregate Bond Coverage), the relative difference between the properties of two laminates is calculated by subtracting the property values:

$$\Delta Property\ that\ is\ reported\ in\ \% = Property\ of\ First\ Ear - Property\ of\ Second\ Ear$$

Differences in ear laminates within the array may correspond to different purchaser/wearer preferences. Returning to Fig. 8, in some embodiments, a property of an ear laminate may be paired with additional functional features 350 in the absorbent article to further meet the purchaser/wearer's preferences. For example, a first ear laminate may comprise greater breathability, softness, extensibility and/or tensile strength, and the first absorbent article may further comprise an additional feature functional feature 350 including but not limited to channels 29, apertured topsheets, enhanced superabsorbent materials or greater absorbency, softer or higher performing leg cuffs (i.e., less leakage), waist features, wetness indicators, secondary fastening systems, and combinations thereof. In nonlimiting examples, the second absorbent article may be void of a functional feature 350 that is present in the first absorbent article, as shown for example in Fig. 8 where the first absorbent article comprises a waist feature 80 and the second absorbent article is void of a waist feature. It is also contemplated that two absorbent articles may comprise functional features that perform similar tasks but are different. For example, the first absorbent article and second absorbent article may each comprise channels 29 but said channels may be shaped differently.

Further, the array 450 may comprise additional absorbent articles, such as a third absorbent article 10c having a third ear laminate 30c. The third ear laminate 30c may comprise any of the features described above, which may be the same as or different from features of the first and second ear laminates.

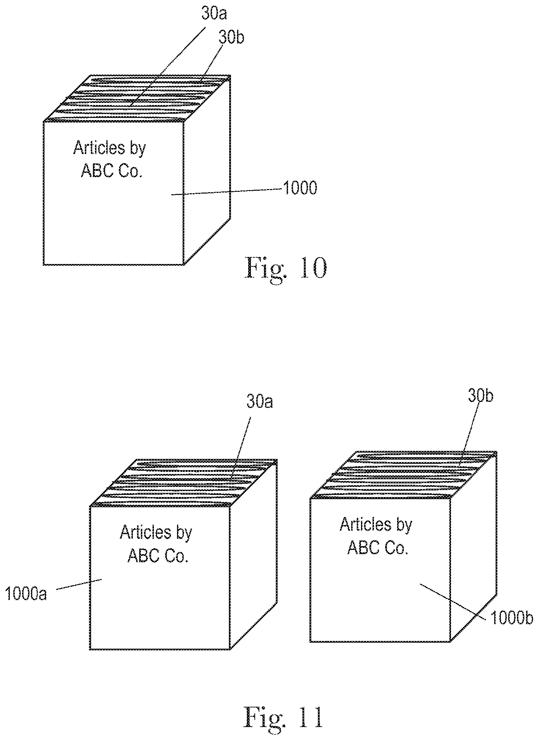

In some embodiments, the first and second absorbent article are disposed in a single package 1000 as shown in Fig. 10. In other embodiments, the first and second absorbent articles are disposed in different packages 1000a, 1000b as shown in Fig. 11. Each package 1000a, 1000b may comprise a plurality of absorbent articles. In nonlimiting examples, the first package 1000a comprises a plurality of first absorbent articles, each of the plurality having a first ear laminate. In further nonlimiting examples, the second package 1000b comprises a plurality of second absorbent articles, each of the plurality having a second ear laminate. The packages may be marketed and/or sold under the same brand name and/or tradename.

The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 110 mm, less than about 105 mm, less than about 100 mm, less than about 95 mm, less than about 90 mm, less than about 85 mm, less than about 80 mm, less than about 78 mm, less than about 76 mm, less than about 74 mm, less than about 72 mm, or less than about 70 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 110 mm, from about 70 mm to about 105 mm, from about 70 mm to about 100 mm, from about 70 mm to about 95 mm, from about 70 mm to about 90 mm, from about 70 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

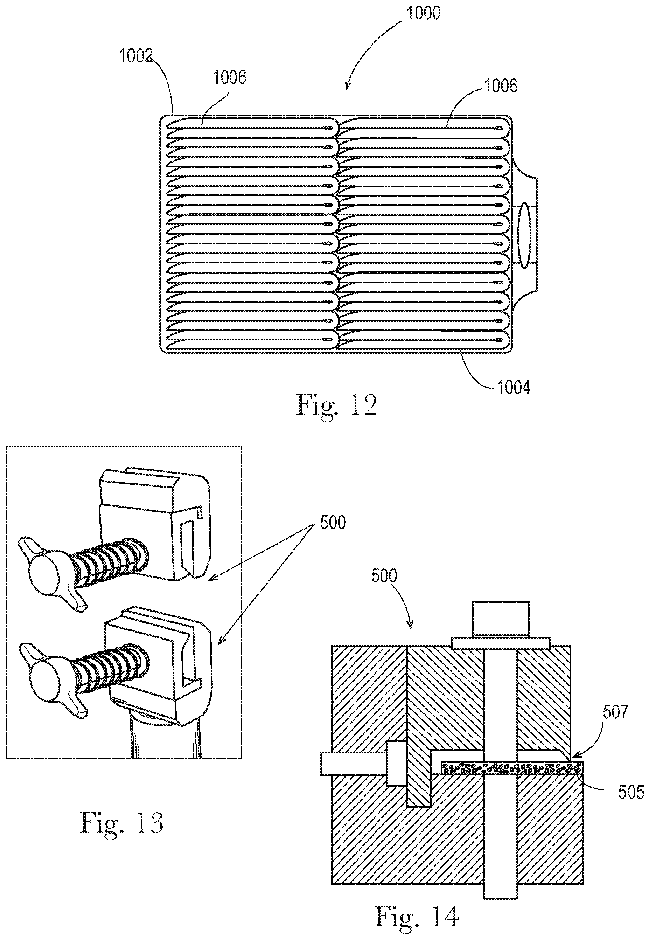

Fig. 12 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

Various combinations may be obtained and fall within the scope of this invention. For instance, the first ear laminate may be softer than the second ear laminate, but the second ear laminate may be more breathable. Each property can be independently varied between ear laminates.

TEST METHODS

Tensile Test Method

The Tensile Test is used to measure the strength of a specimen at a relatively high strain rate that represents product application. The method uses a suitable tensile tester such as an MTS 810, available from MTS Systems Corp., Eden Prairie Minn., or equivalent, equipped with a servohydraulic actuator capable of speeds exceeding 5 m/s after 28 mm of travel, and approaching 6 m/s after 40 mm of travel. The tensile tester is fitted with a 50 lb. force transducer (e.g., available from Kistler North America, Amherst, N.Y. as product code 9712 B50 (50 lb)), and a signal conditioner with a dual mode amplifier (e.g., available from Kistler North America as product code 5010). Grips shown in the Figs. 13 and 14 should be used to secure the specimens during tensile testing. (Fig. 14 is a side view of one of the grips in Fig. 13 with a material 505 to prevent slippage.) The opposing grips 500 may have the same width or different widths as specified.

(a) Grips

The line grips 500 are selected to provide a well-defined gauge and avoid undue slippage. The specimen is positioned such that it has minimal slack between the grips. The apexes 507 of the grips 500 are ground to give good gage definition while avoiding damage or cutting of the specimen. The apexes are ground to provide a radius in the range of 0.5-1.0 mm. A portion of one or both grips 500 may be configured to include a material 505 that reduces the tendency of a specimen to slip, (e.g., a piece of urethane or neoprene rubber having a Shore A hardness of between 50 and 70) as shown in Fig. 14. Six inches wide top and bottom grips are used to clamp the specimen unless specified otherwise.

(b) Tensile Test of Specimen from Absorbent Article

Ears are generally bonded to chassis via thermal or adhesive or similar bonding. Ears should be separated from the chassis in a way that ears are not damaged and performance of the ear is not altered. If the chassis bond is too strong (i.e., ears will be damaged upon removal), then the portion of the chassis joined to the ear should be cut within the chassis material but without damaging the ear. Folded fastening systems (e.g., release tapes covering fastening elements) should be unfolded.

The specimen is clamped in the top grip at a first grip location G1 which is inboard edge 52a of the fastener attachment bond 52 (see Figs. 4-5). The grip line G1 is kept parallel to the longitudinal centerline of the product. If the fastener attachment bond is angled, the specimen is gripped at the center of the bond region and grip line is kept parallel to the longitudinal centerline of the product at the center. The width of the top grip should be equal to the maximum length of the fastener attachment bond 52 (L1) measured parallel to the longitudinal centerline of the article. If, at the G1 position, the length of the specimen is the same as the maximum length of the fastener attachment bond, then any grip width greater than the specimen length at G1 can be used. The specimen is mounted and hung from the top grip. The opposing edge 38 of the specimen is mounted in the bottom grip in relaxed condition. The bottom grip location G2 is adjusted so the specimen is gripped at the outboard edge 35b of the chassis bond. If the chassis bond is curvilinear, the specimen is gripped at the outboard edge of the outermost bond. The bottom grip is greater than the length of the ear at the second grip location, G2. The top and bottom grips are parallel to each other.

The specimen is tested as follows: The vertical distance (perpendicular to the grip line) from the first grip location, G1, to second grip location, G2, is measured to 0.1 mm using ruler and is used as gage length for the test. The specimen is tested at a test speed that provides 9.1 sec$^{-1}$ strain rate with the gage length selected for the specimen. Test speed in mm/second is calculated by multiplying 9.1 sec$^{-1}$ by the gage length in mm. Before testing, 5 mm of slack is put between the grips.

Each specimen is pulled to break. During testing, one of the grips is kept stationary and the opposing grip is moved. The force and actuator displacement data generated during the test are recorded using a MOOG SmarTEST ONE STO03014-205 standalone controller, with the data acquisition frequency set at 1 kHz. The resulting load data may be expressed as load at break in Newton. The Extension (mm) at 5N and at 10N are also recorded. Total of five (5) specimens are run for example. The Average Load at Break and standard deviation, the Average Extension at 5N and standard deviation, and the Average Extension at 10N and standard deviation of at least 4 specimens are recorded. If, standard deviation recorded is higher than 5%, a new set of five specimens is run.

(c) Length Ratio

Per the earlier steps, the grips are positioned at a first grip location and a second grip location. The ratio of the length of the specimen at the second grip position (L2) to the maximum length of bond (L1) is Length Ratio. The respective lengths are measured to 0.1 mm accuracy using the ruler.

Basis Weight Test Method

Each specimen is weighed to within ± 0.1 milligram using a digital balance. Specimen length and width are measured using digital Vernier calipers or equivalent to within ± 0.1 mm. All testing is conducted at 22 ± 2°C and 50 ± 10% relative humidity. Basis weight is calculated using equation below.

$$Basis\ Weight\ \left(\frac{g}{m^2}\right) = \frac{(Weight\ of\ the\ specimen\ in\ grams)}{(Length\ of\ the\ specimen\ in\ meter)(Width\ of\ the\ specimen\ in\ meter)}$$

For calculating the basis weight of a substrate, a total 8 rectilinear specimens at least 10 mm X 25 mm are used.
The average basis weight and standard deviation are recorded.
Nonwoven specimens from ears are obtained as follows. The specimen should be taken from a region having no additional material (i.e., only nonwoven). Each nonwoven layer is separated from the other layers of the ear without damaging or tearing the nonwoven layer. If one continuous nonwoven covers outboard and inboard inelastic regions of the ear, said nonwoven is separated from the inelastic regions and used as the specimen. If the nonwoven layer is inseparable from other ear layers, the specimen is collected from the outboard inelastic region of the ear. If the outboard inelastic region is smaller than the prescribed specimen dimensions or has additional material (other than nonwoven layers), and if the inboard inelastic region has identical nonwovens as the outboard inelastic region, then the specimen (either nonwoven layer or the combination of nonwoven layers) is collected from the inboard inelastic region. If the nonwoven layers in the inelastic region are identical and/or inseparable, then the calculated basis weight of the specimen is divided by the number of nonwoven layers to get the individual nonwoven basis weight.

Hysteresis Test Method

The Hysteresis Test can be used to various specified strain values. The Hysteresis Test utilizes a commercial tensile tester (e.g., from Instron Engineering Corp. (Canton, MA), SINTECH-MTS Systems Corporation (Eden Prairie, MN) or equivalent) interfaced with a computer. The computer is used to control the test speed and other test parameters and for collecting, calculating, and reporting the data. The tests are performed under laboratory conditions of 23°C ± 2°C and relative humidity of 50% ± 2%. The specimens are conditioned for 24 hours prior to testing.
The specimen is cut with a dimension of 10 mm in the intended stretch direction of the ear X 25.4 mm in the direction perpendicular to the intended stretch direction of the ear. A specimen is collected from either an inelastic region or from an elastic region.

Test Protocol

1. Select the appropriate grips and load cell. The grips must have flat surfaces and must be wide enough to grasp the specimen along its full width. Also, the grips should provide adequate force and suitable surface to ensure that the specimen does not slip during testing. The load cell is selected so that the tensile response from the specimen tested is between 25% and 75% of the capacity of the load cell used.
2. Calibrate the tester according to the manufacturer's instructions.
3. Set the distance between the grips (gauge length) at 7 mm.
4. Place the specimen in the flat surfaces of the grips such that the uniform width lies along a direction perpendicular to the gauge length direction. Secure the specimen in the upper grip, let the specimen hang slack, then close the lower grip. Set the slack preload at 5 gram/force. This means that the data collection starts when the slack is removed (at a constant crosshead speed of 13mm/min) with a force of 5 gram force. Strain is calculated based on the adjusted gauge length ($l_{ini}$), which is the length of the specimen in between the grips of the tensile tester at a force of 5 gram force. This adjusted gauge length is taken as the initial specimen length, and it corresponds to a strain of 0%. Percent strain at any point in the test is defined as the change in length relative to the adjusted gauge length, divided by the adjusted gauge length, multiplied by 100.

5(a) First cycle loading: Pull the specimen to the 100% strain at a constant cross head speed of 70 mm/min. Report the stretched specimen length between the grips as $l_{max}$.

5(b) First cycle unloading: Hold the specimen at the 100% strain for 30 seconds and then return the crosshead to its starting position (0% strain or initial sample length, $l_{ini}$) at a constant cross head speed of 70 mm/min. Hold the specimen in the unstrained state for 1 minute.

5(c) Second cycle loading: Pull the specimen to the 100% strain at a constant cross head speed of 70 mm/min.

5(d) Second cycle unload: Next, hold the specimen at the 100% strain for 30 seconds and then return the crosshead to its starting position (i.e. 0% strain) at a constant cross head speed of 70 mm/min.

A computer data system records the force exerted on the sample during the test as a function of applied strain. From the resulting data generated, the following quantities are reported.

    i. Length of specimen between the grips at a slack preload of 5 gram-force ($l_{ini}$) to the nearest 0.001 mm.
    ii. Length of specimen between the grips on first cycle at the 100% strain ($l_{max}$) to the nearest 0.001 mm.
    iii. Length of specimen between the grips at a second cycle load force of 7 gram-force ($l_{ext}$) to the nearest 0.001 mm.
    iv. % Set, which is defined as ($l_{ext}$ - $l_{ini}$) / ($l_{max}$ - $h_{ini}$) * 100% to the nearest 0.01%. The testing is repeated for six separate samples and the average and standard deviation reported.

Air Permeability Test

The air permeability of an ear laminate or substrate (e.g., film, nonwoven, or article component) is determined by measuring the flow rate of standard conditioned air through a test specimen driven by a specified pressure drop. This test is particularly suited to materials having relatively high permeability to gases, such as nonwovens, apertured ear laminates and the like. ASTM D737 is used, modified as follows.

A TexTest FX 3300 instrument or equivalent is used, available from Textest AG, Switzerland, or from Advanced Testing Instruments ATI in Spartanburg SC, USA. The procedures described in the Operating Instructions for the TEXTEST FX 3300 Air Permeability Tester manual for the Air Tightness Test and the Function and Calibration Check are followed. If a different instrument is used, similar provisions for air tightness and calibration are made according to the manufacturer's instructions.

The specimen is tested while in a relaxed state.

The test pressure drop is set to 125 Pascal and the 38.3 cm$^2$ area test head (model FX 3300-5) or equivalent is used. The result is recorded to three significant digits. The average of 5 specimens is calculated and reported as the Air Permeability Value (m$^3$/m$^2$/min).

Bond Dimension Test Method

The Bond Dimension Test is used to measure bond density of a laminate in the various bonding regions. For purposes of this method, a bond is the intentional joining of two or more layers and is the deformed area caused during the bonding process (e.g., the reduced caliper at the site of bonding). It is recognized that in some cases, the deformed area may include one or more apertures.

Specimen Collection

1. Uniform pattern regions: To measure bond density of the bonding region having a uniform pattern, a square specimen of 1 cm$^2$ area is cut from the patterned bonded region of the laminate. Care should be taken to avoid collecting specimen from an adjacent region, if it is different. If specimen collection size of 1 cm$^2$ square is larger than the patterned region, the specimen is collected in the rectangle shape having a 1 cm$^2$ area: the shorter dimension of the patterned region forms one side of the rectangle and the other is selected such a way that rectangle area is 1 cm$^2$.
2. Other regions: To measure bond density of a bonding region without a uniform pattern, identify the plurality of bonds of interest and outline the resulting periphery. The specimen is collected by cutting along the periphery.
3. To the extent bonding regions are not identifiable, the ear may be segmented into three longitudinally extending regions: The first region has a width corresponding to the maximum width between the proximate edge of the ear and edge of the chassis bond closest to the distal edge of the ear. The second region has a width corresponding to the maximum width between the distal edge and the edge of the fastener attachment bond closest to the proximate edge of the ear. The third region has a width that extends between the first and second regions. Each region extends longitudinally for the length of the ear in their respective regions and the lengths may vary in the same manner as the ear's length varies in their respective regions.

Bond Frequency: Bond density by bond frequency is calculated by counting number of bonds on the specimen and dividing the number of bonds by the specimen's area. To the extent that specimen collection creates a partial bond within the specimen area, the partial bond is counted as a fraction equal to the fraction of the area of the bond included within the specimen relative to the area of the whole bond (i.e., the bond prior to cutting the specimen). Bond dimensions are measured to accuracy of 0.01 mm using a microscope and/or imaging software. The dimensions for each bond are used to calculate the bond area as per the mathematical area formula for the given shape of the bond. A total of five specimens are used, and an average bond density by bond frequency is calculated.

Aggregate Bond Coverage: Bond density by aggregate bond coverage is calculated by summing the bond areas for each bond in the specimen and dividing it by the specimen's area. Bond dimensions are measured to accuracy of 0.01 mm using a microscope and/or imaging software. The dimensions for each bond are used to calculate the bond area as per the mathematical area formula for the given shape of the bond. The area of partial bonds inside the specimen are also measured. All bond areas within the specimen are added to calculate aggregate bond area for the specimen and then the aggregate bond area is divided by the area of the specimen to determine aggregate bond coverage. A total of five specimen are used and an average bond density by aggregate bond coverage is calculated.

Softness Test Method

TS7 and TS750 values are measured using an EMTEC Tissue Softness Analyzer ("Emtec TSA") (Emtec Electronic GmbH, Leipzig, Germany) interfaced with a computer running Emtec TSA software (version 3.19 or equivalent). According to Emtec, the TS7 value correlates with the real material softness, while the TS750 value correlates with the felt smoothness/roughness of the material. The Emtec TSA comprises a rotor with vertical blades which rotate on the test sample at a defined and calibrated rotational speed (set by manufacturer) and contact force of 100 mN. Contact between the vertical blades and the test piece creates vibrations, which create sound that is recorded by a microphone within the instrument. The recorded sound file is then analyzed by the Emtec TSA software. TS7 and TS750 values are reported in db $V^2$rms

Sample Preparation

Test samples are prepared by cutting square or circular samples from a finished product. Test samples are cut to a length and width (or diameter if circular) of about 90 mm, and no greater than about 120 mm, in dimension. If the finished product has a discrete section of elasticized region (i.e. elasticized region is shorter in one or more dimensions than nonwoven facing-layers), a set of rectilinear specimens 76 mm $\pm$ 3 mm long in the primary stretch direction, and 100 mm $\pm$ 3 mm wide in the perpendicular direction is cut from the product part, with the elasticized region centered in the rectilinear specimen. Test samples are selected to avoid creases or folds within the testing region. Prepare 8 substantially similar replicate samples for testing. Equilibrate all samples at TAPPI standard temperature and relative humidity conditions (23 °C $\pm$ 2 C° and 50 % $\pm$ 2 %) for at least 1 hour prior to conducting the TSA testing, which is also conducted under TAPPI conditions.

In-Bag Stack Height Test

The in-bag stack height of a package of absorbent articles is determined as follows:

Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within $\pm$ 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 $\pm$ 1 gram-force (8.34 N) on the absorbent article package, which may be achieved by placing a suitable weight on the center of the non-package-contacting top surface of the horizontal sliding plate so that the total mass of the sliding plate plus added weight is 850 $\pm$ 1grams.

Test Procedure

Absorbent article packages are equilibrated at 23 $\pm$ 2 °C and 50 $\pm$ 5 % relative humidity prior to measurement.

The horizontal sliding plate is raised and an absorbent article package is placed centrally under the horizontal sliding plate in such a way that the absorbent articles within the package are in a horizontal orientation (see Fig. 12). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

**Claims**

1. An array (450) of absorbent articles (10) comprising
   a first absorbent article (10a) comprising a topsheet (24), a backsheet (26) and an absorbent core (28) disposed between the topsheet and backsheet, and a first ear laminate (30a) having a first plurality of ultrasonic bonds (46) disposed in a first elasticized region ($306_F$); and
   a second absorbent article (10b) comprising a topsheet, a backsheet and an absorbent core disposed between the topsheet and backsheet, and a second ear laminate (30b) having a second plurality of ultrasonic bonds disposed in a second elasticized region (306s); wherein the first and second ear laminate differ in bond pattern,
   wherein the first plurality of ultrasonic bonds is disposed in a first collective pattern (4000a); and the second plurality of ultrasonic bonds is disposed in a second collective pattern (4000b); wherein the first collective pattern differs from the second collective pattern by average bond spacing, pattern uniformity, bond size, bond shape, bond orientation, aggregate bond area, aggregate pattern shape and combinations thereof.

2. The array of absorbent articles according to claim 1 wherein the first ear laminate has an Average Extension at 10N that is at least 5 % greater than an Average Extension at 10N of the second ear laminate, as measured according to the Tensile Test method described herein.

3. The array of absorbent articles according to claims 1 or 2 wherein the first elasticized region comprises an area that is greater than the area of the second elasticized region.

4. The array of absorbent articles according to any of the preceding claims wherein the first ear laminate comprises a first elastomeric material ($304_F$) and the second ear laminate comprises a second elastomeric material ($304_S$), wherein the first and second elastomeric materials differ by basis weight, material type, composition and combinations thereof.

5. The array of absorbent articles of any of the preceding claims wherein the first ear laminate comprises a primary nonwoven (360) and the second ear laminate comprises a secondary nonwoven (370), wherein the primary nonwoven and the secondary nonwoven differ by one of the group consisting of layer configuration, fiber composition, calendar bond area or calendar bond shape, basis weight, and combinations thereof.

6. The array according to claim 5 wherein the first collective pattern corresponds to a first user characteristic and the second pattern corresponds to second user characteristic, wherein the first and second user characteristics are different and are each selected from the group consisting of: intended wearer gender, intended wearer age and/or development stage, absorbency needs, fit preferences, and combinations thereof.

7. The array of absorbent articles according to any of the preceding claims wherein the first ear laminate comprises a TS7 softness value that is at least 10% less than a TS7 softness value of the second ear laminate, as measured according to the Softness Test method described herein.

8. The array of absorbent articles of claim 7 wherein the first ear laminate comprises one of the group consisting of a crimped fiber spunbond nonwoven web, a meltblown nonwoven web, a softness additive, a carded nonwoven web, and combinations thereof.

9. The array of absorbent articles according to any of the preceding claims wherein the first ear laminate comprises first Average Load at Break and the second ear laminate comprises a second Average Load at Break, wherein the first Average Load at Break is at least 4% greater than the second Average Load at Break.

10. The array of absorbent articles according to any of the preceding claims wherein the first ear laminate comprises a first garment-facing nonwoven and a first body-facing nonwoven, wherein at least one of the first garment-facing and first body-facing nonwovens comprises a basis weight of about 20 gsm or less.

11. The array of absorbent articles according to any of the preceding claims wherein the first absorbent article and second absorbent article have different sizes.

12. The array of absorbent articles according to any of the preceding claims wherein a single package (1000) comprises the first and second absorbent articles.

13. The array of absorbent articles according to any of claims 1-11 wherein a first package (1000a) comprises the first absorbent article and a second package (1000b) comprises the second absorbent article, and wherein the first and second packages comprise a common brand name.

14. The array of absorbent articles according to any of the preceding claims wherein the first absorbent article comprises a functional feature (350) and the second absorbent article is void of said functional feature.

**Patentansprüche**

1. Anordnung (450) von Absorptionsartikeln (10), umfassend
einen ersten Absorptionsartikel (10a), der eine Oberschicht (24), eine Unterschicht (26) und einen Absorptionskern (28), der zwischen der Oberschicht und der Unterschicht angeordnet ist, und ein erstes Ohrlaminat (30a) umfasst, das eine erste Vielzahl von Ultraschallbindungen (46) aufweist, die in einem ersten elastifizierten Bereich ($306_F$) angeordnet sind; und
einen zweiten Absorptionsartikel (10b), der eine Oberschicht, eine Unterschicht und einen Absorptionskern, der zwischen der Oberschicht und der Unterschicht angeordnet ist, und ein zweites Ohrlaminat (30b) umfasst, das eine zweite Vielzahl von Ultraschallbindungen aufweist, die in einem zweiten elastifizierten Bereich (306s) angeordnet sind;
wobei sich das erste und das zweite Ohrlaminat hinsichtlich des Bindungsmusters unterschieden,
wobei die erste Vielzahl von Ultraschallbindungen in einem ersten Sammelmuster (4000a) angeordnet ist; und die zweite Vielzahl von Ultraschallbindungen in einem zweiten Sammelmuster (4000b) angeordnet ist; wobei sich das erste Sammelmuster von dem zweiten Sammelmuster durch durchschnittlichen Bindungsabstand, Mustergleichmäßigkeit, Bindungsgröße, Bindungsform, Bindungsorientierung, Gestamtbindungsfläche, Gesamtmusterform und Kombinationen davon unterscheidet.

2. Anordnung von Absorptionsartikeln nach Anspruch 1, wobei das erste Ohrlaminat bei 10 N eine mittlere Ausdehnung aufweist, die mindestens 5 % größer ist als eine mittlere Ausdehnung des zweiten Ohrlaminats bei 10 N, gemessen gemäß dem hierin beschriebenen Zugprüfverfahren.

3. Anordnung von Absorptionsartikeln nach einem der Ansprüche 1 oder 2, wobei der erste elastifizierte Bereich eine Fläche aufweist, die größer ist als die Fläche des zweiten elastifizierten Bereichs.

4. Anordnung von Absorptionsartikeln nach einem der vorstehenden Ansprüche, wobei das erste Ohrlaminat ein erstes Elastomermaterial ($304_F$) umfasst und das zweite Ohrlaminat ein zweites Elastomermaterial (304s) umfasst, wobei sich das erste und das zweite Elastomermaterial durch das Flächengewicht, den Materialtyp, die Zusammensetzung und Kombinationen davon unterscheiden.

5. Anordnung von Absorptionsartikeln nach einem der vorstehenden Ansprüche, wobei das erste Ohrlaminat ein primäres Vlies (360) umfasst und das zweite Ohrlaminat ein sekundäres Vlies (370) umfasst, wobei sich das primäre Vlies und das sekundäre Vlies durch eines aus der Gruppe bestehend aus Schichtkonfiguration, Faserzusammensetzung, Kalenderklebefläche oder Kalenderbindungsform, Flächengewicht und Kombinationen davon unterscheiden.

6. Anordnung nach Anspruch 5, wobei das erste Sammelmuster einer ersten Benutzereigenschaft entspricht und das zweite Muster einer zweiten Benutzereigenschaft entspricht, wobei die erste und zweite Benutzereigenschaft unterschiedlich sind und jeweils aus der Gruppe ausgewählt sind, bestehend aus: Geschlecht des beabsichtigten Trägers, Alter und/oder Entwicklungsstadium des beabsichtigten Trägers, Absorptionsanforderungen, Passform-Präferenzen und Kombinationen davon.

7. Anordnung von Absorptionsartikeln nach einem der vorstehenden Ansprüche, wobei das erste Ohrlaminat einen TS7-Weichheitswert aufweist, der mindestens 10 % kleiner als ein TS7-Weichheitswert des zweiten Ohrlaminats ist, gemessen gemäß dem hierin beschriebenen Weichheitstestverfahren.

8. Anordnung von Absorptionsartikeln nach Anspruch 7, wobei das erste Ohrlaminat eines aus der Gruppe bestehend aus einer Kräuselfaserspinnvliesbahn aus gekräuselten Fasern, einem schmelzgeblasenen Vlies, einem Weichmacherzusatzstoff, einem kardierten Vlies und Kombinationen davon umfasst.

9. Anordnung von Absorptionsartikeln nach einem der vorstehenden Ansprüche, wobei das erste Ohrlaminat eine erste durchschnittliche Bruchlast umfasst und das zweite Ohrlaminat eine zweite durchschnittliche Bruchlast umfasst, wobei die erste durchschnittliche Bruchlast mindestens 4 % größer ist als die zweite durchschnittliche Bruchlast.

10. Anordnung von Absorptionsartikeln nach einem der vorstehenden Ansprüche, wobei das erste Ohrlaminat ein erstes, dem Kleidungsstück zugewandtes Vlies und ein erstes, dem Körper zugewandtes Vlies umfasst, wobei mindestens eines von dem ersten, dem Kleidungsstück zugewandten und dem ersten, dem Körper zugewandten Vlies ein Flächengewicht von 20 Gramm pro Quadratmeter oder weniger umfasst.

11. Anordnung von Absorptionsartikeln nach einem der vorstehenden Ansprüche, wobei der erste Absorptionsartikel und der zweite Absorptionsartikel unterschiedliche Größen aufweisen.

12. Anordnung von Absorptionsartikeln nach einem der vorstehenden Ansprüche, wobei eine Einzelverpackung (1000) den ersten und den zweiten Absorptionsartikel umfasst.

13. Anordnung von Absorptionsartikeln nach einem der Ansprüche 1 bis 11, wobei eine erste Verpackung (1000a) den ersten Absorptionsartikel umfasst und eine zweite Verpackung (1000b) den zweiten Absorptionsartikel umfasst und wobei die erste und die zweite Verpackung einen gemeinsamen Markennamen umfassen.

14. Anordnung von Absorptionsartikeln nach einem der vorstehenden Ansprüche, wobei der erste Absorptionsartikel ein funktionelles Merkmal (350) umfasst und der zweite Absorptionsartikel frei von dem funktionellen Merkmal ist.

**Revendications**

1. Réseau (450) d'articles absorbants (10) comprenant
   un premier article absorbant (10a) comprenant une feuille de dessus (24), une feuille de fond (26) et une âme absorbante (28) disposée entre la feuille de dessus et la feuille de fond, et un premier stratifié à patte (30a) ayant une première pluralité de liaisons par ultrasons (46) disposées dans une première région élastifiée ($306_F$) ; et
   un second article absorbant (10b) comprenant une feuille de dessus, une feuille de fond et une âme absorbante disposée entre la feuille de dessus et la feuille de fond, et un second stratifié à patte (30b) ayant une seconde pluralité de liaisons par ultrasons disposées dans une seconde région élastifiée (306s) ;
   dans lequel le premier et le second stratifié à patte diffèrent dans le motif de liaison,

dans lequel la première pluralité de liaisons par ultrasons est disposée dans un premier motif collectif (4000a) ; et la seconde pluralité de liaisons par ultrasons est disposée dans un second motif collectif (4000b) ; dans lequel le premier motif collectif diffère du second motif collectif par l'espacement moyen de liaison, l'uniformité de motif, la taille de liaison, la forme de liaison, l'orientation de liaison, l'aire globale de liaison, la forme globale de motif et des combinaisons de ceux-ci.

2. Réseau d'articles absorbants selon la revendication 1 dans lequel le premier stratifié à patte a une extension moyenne à 10 N qui est au moins 5 % supérieure à une extension moyenne à 10 N du second stratifié à patte, telle que mesurée selon le procédé d'essai de traction décrit ici.

3. Réseau d'articles absorbants selon les revendications 1 ou 2 dans lequel la première région élastifiée comprend une aire qui est supérieure à l'aire de la seconde région élastifiée.

4. Réseau d'articles absorbants selon l'une quelconque des revendications précédentes dans lequel le premier stratifié à patte comprend un premier matériau élastomère ($304_F$) et le second stratifié à patte comprend un second matériau élastomère (304s), dans lequel les premier et second matériaux élastomères diffèrent par le poids de base, le type de matériau, la composition et des combinaisons de ceux-ci.

5. Réseau d'articles absorbants selon l'une quelconque des revendications précédentes dans lequel le premier stratifié à patte comprend un nontissé primaire (360) et le second stratifié à patte comprend un nontissé secondaire (370), dans lequel le nontissé primaire et le nontissé secondaire diffèrent par l'un du groupe constitué de configuration de couche, de composition de fibre, d'aire de liaison calendaire ou de forme de liaison calendaire, de masse surfacique, et des combinaisons de ceux-ci.

6. Réseau selon la revendication 5 dans lequel le premier motif collectif correspond à une première caractéristique d'utilisateur et le second motif correspond à une seconde caractéristique d'utilisateur, dans lequel les première et seconde caractéristiques d'utilisateur sont différentes et sont chacune choisies dans le groupe constitué de : sexe du porteur prévu, âge et/ou stade de développement du porteur prévu, besoins d'absorbance, préférences d'ajustement, et des combinaisons de ceux-ci.

7. Réseau d'articles absorbants selon l'une quelconque des revendications précédentes dans lequel le premier stratifié à patte comprend une valeur de douceur TS7 qui est au moins 10 % inférieur à une valeur de douceur TS7 du second stratifié à patte, telle que mesurée selon le procédé de test de douceur décrit ici.

8. Réseau d'articles absorbants selon la revendication 7 dans lequel le premier stratifié à patte comprend l'un du groupe constitué d'un voile non tissé filé-lié de fibres frisées, d'un voile non tissé soufflé en fusion, d'un additif de douceur, d'un voile non tissé cardé, et des combinaisons de ceux-ci.

9. Réseau d'articles absorbants selon l'une quelconque des revendications précédentes dans lequel le premier stratifié à patte comprend une première charge moyenne de rupture et le second stratifié à patte comprend une seconde charge moyenne de rupture, dans lequel la première charge moyenne de rupture est au moins 4 % supérieure à la seconde charge moyenne de rupture.

10. Réseau d'articles absorbants selon l'une quelconque des revendications précédentes dans lequel le premier stratifié à patte comprend un premier nontissé tourné vers le vêtement et un premier nontissé tourné vers le corps, dans lequel au moins l'un des nontissés premier tourné vers le vêtement et premier tourné vers le corps comprend une masse surfacique d'environ 20 g/m$^2$ ou moins.

11. Réseau d'articles absorbants selon l'une quelconque des revendications précédentes dans lequel le premier article absorbant et le second article absorbant ont des tailles différentes.

12. Réseau d'articles absorbants selon l'une quelconque des revendications précédentes dans lequel un seul emballage (1000) comprend les premier et second articles absorbants.

13. Réseau d'articles absorbants selon l'une quelconque des revendications 1 à 11 dans lequel un premier emballage (1000a) comprend le premier article absorbant et un second emballage (1000b) comprend le second article absorbant, et dans lequel les premier et second emballages comprennent un nom de marque commun.

**14.** Réseau d'articles absorbants selon l'une quelconque des revendications précédentes dans lequel le premier article absorbant comprend une caractéristique fonctionnelle (350) et le second article absorbant est vide de ladite caractéristique fonctionnelle.

Fig. 1

Fig. 2

Fig. 3

EP 3 538 046 B1

Fig. 4

Fig. 5

Fig. 6

EP 3 538 046 B1

400a  402  400b  404  46  30  44  4000

46

400c

400d

50

46  52

400e

Fig. 7

Fig. 8

Fig. 9

30a
30b

Articles by
ABC Co.

1000

Fig. 10

30a
30b

Articles by
ABC Co.

1000a

Articles by
ABC Co.

1000b

Fig. 11

Fig. 12

Fig. 13

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120238980 A
- US 6120487 A
- US 20130082418 A
- US 5167897 A
- US 5993432 A
- US 3849241 A, Buntin
- US 3338992 A
- US 3692613 A
- US 3802817 A
- US 4405297 A
- US 5665300 A
- US 3860003 A
- US 5151092 A
- US 5221274 A
- US 5554145 A
- US 5569234 A
- US 5580411 A
- US 6004306 A
- US 5607760 A
- US 5609587 A
- US 5635191 A
- US 5643588 A
- US 4892536 A
- US 4990147 A
- US 5037416 A
- US 5269775 A
- US 4610678 A
- US 4673402 A
- US 4834735 A
- US 4888231 A
- US 5137537 A
- US 5147345 A
- US 5342338 A
- US 5260345 A
- US 5387207 A
- US 5397316 A
- US 491642
- US 15232901 B
- WO 9516746 A
- US 5865823 A
- US 5571096 A
- US 6107537 A
- US 8835709 B
- US 8618350 B
- US 6410129 B
- US 7819853 B
- US 8795809 B
- US 7806883 B
- US 6677258 B
- US 20090258210 A
- US 9533067 B
- US 62374010
- US 62419515 B
- US 3848594 A
- US 4662875 A
- US 4846815 A
- US 4894060 A
- US 4946527 A
- US 6432098 B
- US 62134622
- US 14077708 B
- US 8939957 B
- US 7435243 B
- US 8062279 B
- US 490543
- US 14533472 B
- US 62134622 B